# EUROPEAN PATENT APPLICATION

(11) **EP 3 973 968 A2**
(43) Date of publication of application: **30.03.2022**
(21) Application number: 21189242.7
(22) Date of filing: 10.11.2016
(51) Int. Cl.: A61K 35/15, A61K 38/44, C12N 5/0786, C12N 5/078, C12N 15/00, A61P 35/00

(54) **METHOD OF MODIFYING MACROPHAGE DIFFERENTIATION AND IMMUNITY**

(30) Priority: 11.11.2015 US 201562253836 P
(62) Divisional of application: 16864994.5
(71) Applicant: Zhu, Zhenglun, Newton, MA 02459 (US)
(72) Inventor: Zhu, Zhenglun, Newton, MA 02459 (US)
(74) Representative: Purdylucey Intellectual Property

(57) **Abstract**

A method of treating a cancer, the method comprising: providing a genetically modified macrophage or monocyte that contains a nucleic acid sequence encoding a Hom-1 polypeptide or a fragment thereof that contains the Hom-1 homeobox domain, wherein the nucleic acid sequence is operably linked to a heterologous promoter and the modified macrophage or monocyte expresses the Hom-1 polypeptide or the fragment thereof; and administering the modified macrophage or monocyte to a subject with a cancer.

SEQUENCE LISTING

<110>  Zu, Zhenglun

<120>  METHOD OF MODIFYING MACROPHAGE DIFFERENTIATION AND IMMUNITY

<130>  218008-0005PCT

<150>  US 62/253,836
<151>  2015-11-11

<160>  2

<170>  PatentIn version 3.5

<210>  1
<211>  2428
<212>  DNA
<213>  Homo sapiens

<220>
<221>  CDS
<222>  (12)..(788)
<223>  Hom-1

<400>  1
acctggccgc c atg cgc ctc tcc tcc tcc cca cct cgt ggc ccg cag cag        50
             Met Arg Leu Ser Ser Ser Pro Pro Arg Gly Pro Gln Gln
             1               5                   10

ctc tcc agc ttt ggc tcc gtg gac tgg ctc tcc cag agc agc tgc tca        98
Leu Ser Ser Phe Gly Ser Val Asp Trp Leu Ser Gln Ser Ser Cys Ser
    15                  20                  25

ggg ccg acc cac acc ccc agg cct gcc gac ttc tcc ctg ggg agc ctc       146
Gly Pro Thr His Thr Pro Arg Pro Ala Asp Phe Ser Leu Gly Ser Leu
30                  35                  40                  45

cct ggc cca ggc cag aca tcc ggc gcc cgg gag ccc cct cag gcc gtc       194
Pro Gly Pro Gly Gln Thr Ser Gly Ala Arg Glu Pro Pro Gln Ala Val
                50                  55                  60

agc atc aag gag gcc gcc ggg tcc tca aat ctg cct gcg ccg gag agg       242
Ser Ile Lys Glu Ala Ala Gly Ser Ser Asn Leu Pro Ala Pro Glu Arg
            65                  70                  75

acc atg gcc ggg ttg agt aag gag cca aat acc ttg cgg gcc ccc cgt       290
Thr Met Ala Gly Leu Ser Lys Glu Pro Asn Thr Leu Arg Ala Pro Arg
        80                  85                  90

gtc cgc aca gcc ttc acc atg gag cag gtc cgc acc ttg gag ggc gtc       338
Val Arg Thr Ala Phe Thr Met Glu Gln Val Arg Thr Leu Glu Gly Val
    95                  100                 105

ttc cag cac cac cag tac ctg agc cct ctg gag cgg aag agg ctg gcc       386
Phe Gln His His Gln Tyr Leu Ser Pro Leu Glu Arg Lys Arg Leu Ala
110                 115                 120                 125

agg gag atg cag ctc tca gag gtc cag ata aaa acc tgg ttt cag aat       434
Arg Glu Met Gln Leu Ser Glu Val Gln Ile Lys Thr Trp Phe Gln Asn
                130                 135                 140

cgc cgc atg aaa cac aaa cgg caa atg cag gac ccc cag ctg cac agc     482
Arg Arg Met Lys His Lys Arg Gln Met Gln Asp Pro Gln Leu His Ser
            145                 150                 155

ccc ttc tcg ggg tct ctc cat gcg ccc cca gct ttc tac tca acg tct     530
Pro Phe Ser Gly Ser Leu His Ala Pro Pro Ala Phe Tyr Ser Thr Ser
        160                 165                 170

tct ggc ctt gcc aat ggc ctg cag ctg ctg tgc cct tgg gca ccc ctg     578
Ser Gly Leu Ala Asn Gly Leu Gln Leu Leu Cys Pro Trp Ala Pro Leu
    175                 180                 185

tcc ggg ccc cag gct ctg atg ctg ccc cct ggc tcc ttc tgg ggt ctc     626
Ser Gly Pro Gln Ala Leu Met Leu Pro Pro Gly Ser Phe Trp Gly Leu
190                 195                 200                 205

tgc caa gtg gca caa gag gcc ctg gca tct gcg gga gct tcc tgc tgc     674
Cys Gln Val Ala Gln Glu Ala Leu Ala Ser Ala Gly Ala Ser Cys Cys
                210                 215                 220

ggg cag cct ctg gcg tcc cac ccc cct acc cca ggc cgg cct tcg ctg     722
Gly Gln Pro Leu Ala Ser His Pro Pro Thr Pro Gly Arg Pro Ser Leu
            225                 230                 235

gga cca gcc ctg tcc acg ggg ccc cgg ggc ctg tgt gct atg cca cag     770
Gly Pro Ala Leu Ser Thr Gly Pro Arg Gly Leu Cys Ala Met Pro Gln
        240                 245                 250

acg ggg gat gca ttt tga ggaggcacct ctgactccca cactcgcggt     818
Thr Gly Asp Ala Phe
    255

cttgctgatc gcacctggct cctacctgga ggactcagtt gttctgttta catcctggtg     878

gcacctctca ccctgaccca cacaaaggtt ctggagatta ctggagaata tatataaata     938

tatatatgta cgtatatatg taaatacaca tatacgtata tataaatata tatatacata     998

tgtgtgtgta tatatatata tatttttttt tttttttttt tttttgagac ggagtgttgc     1058

tctgtcaccc aggctggagt gcaatgacgc aatctcggct cactgcaacc tccgcctcct     1118

gggttcaagc gattctccag cctcagcctc ccgagtagct gggattacag acacccgcca     1178

ccacgcccgg ctaatttttt ctatttttag tagaaatggg gtttcaccat gttagccagg     1238

ctggtctcaa actcctgacc ctgtgatccg cccgcctcgg cctcccaaag tgctgggatt     1298

acaggcatga gccactgcac ccggccctga gaatatattt attaaagcca cctcttcact     1358

gaaagttacc gaaagagtcg gtttaggaag gaaacgaagg gtcagtgaac agagtcaaat     1418

gcagaagtgg gcttgtcatg ggtagggctt tcggcgtacg ataaaaggat catttgtttt     1478

ttaaaagggg ttggaaaaac tggttttcca gttggaaaca gtaaaggttg taagctttgt     1538

gtgtacaaaa gaaaacaggg aatgcaggtg tgtttatagc gttgtggttc aagtccctct     1598

taacaagaac tccaaagctg gaaagcagga gggaacaaag gtgaacatga aggcgaggat     1658

gctggggccc tgcagtgcgc tctaggctgt gcgtgagccg ggactgtacc cacagcttgc     1718

tgagggctgc tcttcttggg ccagggaaag cagggcagcc gggacctgcg gctgtgcctg     1778

gactgaagct gtcccgcagg tccccaccct ccaacacgtg ctcacctgtc cccctcctcg 1838

cagcagcctc gggacaaaac aatgactcaa ggacagcact tctcgcagaa ggtctggaag 1898

tgcccagaat gggaggcacg gaagcccctc ccggggagga ctcccgcgtt gatggaccgt 1958

tcttggtgca gactcctgac tgcgtgcatg aaacctgaga caagtgcaat tccttccatg 2018

tcgccccaga gtgcccagga ggcaggcagt gcggggtgcc caggcagacg ggttcagcct 2078

gcagaactgg aggcgacctg tgaaacccac ccgggcaccc caacaggaac agaagcgtgg 2138

tcctgcggct gcgtccccag cgagtttcac tttccccttg ctcgtttctc ccttgttgta 2198

agtgtttaca actggcatgt gcttttaaac gtcaggtaag aggggaacag ctgctgtaca 2258

tcgtcctggc gagtgacaat gtgacagaag cctgggcgag gccctcggag ggcagcagct 2318

ggacaggggc tactgggttt ggcctggaca gcactgattt gtggatgtgg atgggggcac 2378

gttgtccgtg ataaaagtac aagtgcccct cacaaaaaaa aaaaaaaaaa 2428

<210> 2
<211> 258
<212> PRT
<213> Homo sapiens

<400> 2

Met Arg Leu Ser Ser Ser Pro Pro Arg Gly Pro Gln Gln Leu Ser Ser
1               5                   10                  15

Phe Gly Ser Val Asp Trp Leu Ser Gln Ser Ser Cys Ser Gly Pro Thr
            20                  25                  30

His Thr Pro Arg Pro Ala Asp Phe Ser Leu Gly Ser Leu Pro Gly Pro
        35                  40                  45

Gly Gln Thr Ser Gly Ala Arg Glu Pro Pro Gln Ala Val Ser Ile Lys
    50                  55                  60

Glu Ala Ala Gly Ser Ser Asn Leu Pro Ala Pro Glu Arg Thr Met Ala
65                  70                  75                  80

Gly Leu Ser Lys Glu Pro Asn Thr Leu Arg Ala Pro Arg Val Arg Thr
                85                  90                  95

Ala Phe Thr Met Glu Gln Val Arg Thr Leu Glu Gly Val Phe Gln His
            100                 105                 110

His Gln Tyr Leu Ser Pro Leu Glu Arg Lys Arg Leu Ala Arg Glu Met
        115                 120                 125

Gln Leu Ser Glu Val Gln Ile Lys Thr Trp Phe Gln Asn Arg Arg Met
    130                 135                 140

Lys His Lys Arg Gln Met Gln Asp Pro Gln Leu His Ser Pro Phe Ser
145                 150                 155                 160

Gly Ser Leu His Ala Pro Pro Ala Phe Tyr Ser Thr Ser Ser Gly Leu
                165                 170                 175

Ala Asn Gly Leu Gln Leu Leu Cys Pro Trp Ala Pro Leu Ser Gly Pro
            180                 185                 190

Gln Ala Leu Met Leu Pro Pro Gly Ser Phe Trp Gly Leu Cys Gln Val
        195                 200                 205

Ala Gln Glu Ala Leu Ala Ser Ala Gly Ala Ser Cys Cys Gly Gln Pro
    210                 215                 220

Leu Ala Ser His Pro Pro Thr Pro Gly Arg Pro Ser Leu Gly Pro Ala
225                 230                 235                 240

Leu Ser Thr Gly Pro Arg Gly Leu Cys Ala Met Pro Gln Thr Gly Asp
                245                 250                 255

Ala Phe

## Description

### CROSS-REFERENCE TO RELATED APPLICATION

This application claims priority to U.S. Provisional Application No. 62/253,836, filed on November 11, 2015, the content of which is hereby incorporated by reference in its entirety.

### BACKGROUND

The role of immunity in oncogenesis has been increasingly appreciated. Macrophages are executors of both innate and adaptive immunity and have been recognized as key components of tumors and their microenvironment. Extensive investigations suggested the role of tumor-associated macrophages (TAMs) in the growth, invasion and metastasis of nearly all tumors. Derived from circulating monocytes, TAMs display a broad spectrum of phenotypes, ranging from the M1-like phenotype in early stages of selected tumors to the M2-like phenotype in most advanced tumors. Consistent with its role in promoting tumorigenesis, the M2-like TAMs display the characteristic phenotype of elevated expression of IL-10, IL4, MMP, and VEGF, but decreased expression of pro-inflammatory cytokines and cytotoxic iNOs and ROIs, which are implicated in tumoricidal activities. Besides its intrinsic function in promoting tumorigenesis, TAMs also contribute to the suppression of anti-tumor immunity by alternating T-cell responses and balance in the tumor microenvironment. The functional plasticity of TAMs was well recognized. However, the effectiveness and potential application of targeting TAMs in tumor treatment has been restricted by the limited understanding of how TAMs plasticity is controlled by cell intrinsic factors.

### SUMMARY

In one aspect, provided herein is a method of treating a cancer. The method comprising: providing a genetically modified macrophage or monocyte that contains a nucleic acid sequence encoding a Hom-1 polypeptide or a fragment thereof that contains the Hom-1 homeobox domain, wherein the nucleic acid sequence is operably linked to a heterologous promoter and the modified macrophage or monocyte expresses the Hom-1 polypeptide or the fragment thereof; and administering the modified macrophage or monocyte to a subject with a cancer. In one embodiment, the modified macrophage or monocyte is generated by introducing an exogenous expression construct into a macrophage or monocyte derived from the subject or another subject. The modified macrophage exhibits an M1 phenotype. The method can further include, prior to the administering step, detecting a lower level of Hom-1 expression in a tumor-associated macrophage in the subject as compared to a control.

In another aspect, described herein is a method of treating a cancer that includes contacting a macrophage or monocyte with one or more agents that induce expression of Hom-1, whereby the expression level of endogenous Hom-1 in the macrophage or monocyte is higher than before the contacting step; and administering the thus contacted macrophage or monocyte to a subject with a cancer.

Also described herein is a method of treating a cancer that comprises contacting a macrophage or monocyte with an agent that induces the expression of an M1 gene or an agent that inhibits the expression of an M2 gene, whereby a macrophage that exhibits an M1-phenotype is generated; and administering the thus generated macrophage to a subject with cancer.

In yet another aspect, described herein is a method of identifying a cancer in a subject. The method includes detecting the expression level of a gene in a macrophage from the microenvironment of a tissue area suspected of being a cancer, the gene being a Hom-1 gene, M1 gene, or M2 gene, wherein detecting (i) a lower level of the Hom-1 gene or M1 gene or (ii) a higher expression level of the M2 gene as compared to a corresponding control level indicates that the suspected tissue area is a cancer or is at risk of becoming a cancer.

A method of identifying a candidate compound for treating a cancer is also described in this disclosure. The method includes contacting a test cell with a test compound, wherein the test cell is a macrophage or monocyte; detecting in the test cell the expression level of (i) Hom-1, (ii) a reporter gene operably linked to a Hom-1 promoter, (iii) an M1 gene, or (iv) an M2 gene; and selecting a test compound that alters the expression level as compared to a corresponding control level, wherein the selected compound is a candidate compound for treating cancer. In one embodiment, the method can further include contacting a protumor macrophage with the selected compound and assaying the thus contacted protumor macrophage for an anti-tumor activity. The protumor macrophage can be a tumor-associated macrophage or M2 macrophage. The contacting step can be performed with the test cell in a co-culture containing a cancer sample. The test cell can be selected from an M1 macrophage, M2 macrophage, tumor-associated macrophage, tissue macrophage, and monocyte-derived macrophage.

In one aspect, described herein is a method of identifying a candidate compound for treating a cancer that includes: providing a co-culture containing a test cell and a cancer sample, wherein the test cell is a macrophage or monocyte; adding a test compound to the co-culture; and selecting a test compound that, as compared to a control, (i) inhibits the cancer sample, (ii) increases the expression level of Hom-1 or a reporter gene operably linked to a Hom-1 promoter in the test cell, (iii) increases the expression of an M1 gene in the test cell, (iv) decreases the expression of an M2 gene in the test cell, or (v) inhibits a significant decrease of the expression level of Hom-1 or a reporter gene operably linked to a Hom-1 promoter in the test cell; wherein the selected compound is a candidate compound for treating cancer. In one embodiment, the cancer sample is a cancer tissue sample. In the co-culture, the test cell and cancer sample can be in direct contact or in indirect contact with each other. The test cell can be selected from an M1 macrophage, M2 macrophage, tumor-associated macrophage, tissue macrophage, and monocyte-derived macrophage.

In another aspect, described herein is a method of identifying a candidate compound for treating a cancer that includes: providing a cancer tissue sample, contacting the tissue sample with a test compound, and selecting a test compound that, as compared to a control, (i) inhibits the tissue sample, (ii) increases the expression level of Hom-1 in a tumor-associated macrophage or monocyte in the tissue sample, (iii) increases the expression of an M1 gene in a tumor-associated macrophage or monocyte in the tissue sample, (iv) decreases the expression of an M2 gene in a tumor-associated macrophage or monocyte in the tissue sample, or (v) inhibits a significant decrease of the expression level of Hom-1 in a tumor-associated macrophage or monocyte in the tissue sample; wherein the selected compound is a candidate compound for treating cancer.

The details of one or more embodiments are set forth in the description below. Other features, objects, and advantages of the embodiments will be apparent from the description and from the claims.

### DETAILED DESCRIPTION

It was surprisingly discovered that Hom-1 expression in TAMs is significantly decreased in comparison with macrophages isolated from normal tissues. It was further discovered that, also surprisingly, increasing the expression of Hom-1 in TAMs converted them to M1-like macrophages with tumoricidal activities.

Hom-1, a human homeobox transcriptional factor, is an antagonist of the canonical Wnt signaling. A nucleic acid sequence of Hom-1 (SEQ ID NO:1) and the amino acid sequence (SEQ ID NO:2) it encodes are shown below:

acctggccgc catgcgcctc tcctcctccc cacctcgtgg cccgcagcag ctctccagct
ttggctccgt ggactggctc tcccagagca gctgctcagg gccgacccac acccccaggc
ctgccgactt ctccctgggg agcctccctg gcccaggcca gacatccggc gcccgggagc
cccctcaggc cgtcagcatc aaggaggccg ccgggtcctc aaatctgcct gcgccggaga
ggaccatggc cgggttgagt aaggagccaa ataccttgcg ggccccccgt gtccgcacag
ccttcaccat ggagcaggtc cgcaccttgg agggcgtctt ccagcaccac cagtacctga
gccctctgga gcggaagagg ctggccaggg agatgcagct ctcagaggtc cagataaaaa
cctggtttca gaatcgccgc atgaaacaca aacggcaaat gcaggacccc cagctgcaca
gccccttctc ggggtctctc catgcgcccc cagctttcta ctcaacgtct tctggccttg
ccaatggcct gcagctgctg tgcccttggg cacccctgtc cgggccccag gctctgatgc
tgccccctgg ctccttctgg ggtctctgcc aagtggcaca agaggccctg gcatctgcgg
gagcttcctg ctgcgggcag cctctggcgt cccacccccc taccccaggc cggccttcgc
tgggaccagc cctgtccacg gggccccggg gcctgtgtgc tatgccacag acgggggatg
cattttgagg aggcacctct gactcccaca ctcgcggtct tgctgatcgc acctggctcc
tacctggagg actcagttgt tctgtttaca tcctggtggc acctctcacc ctgacccaca
caaaggttct ggagattact ggagaatata tataaatata tatatgtacg tatatatgta
aatacacata tacgtatata taaatatata tatacatatg tgtgtgtata tatatatata
tttttttttt tttttttttt tttgagacgg agtgttgctc tgtcacccag gctggagtgc
aatgacgcaa tctcggctca ctgcaacctc cgcctcctgg gttcaagcga ttctccagcc
tcagcctccc gagtagctgg gattacagac acccgccacc acgcccggct aattttttct
atttttagta gaaatggggt ttcaccatgt tagccaggct ggtctcaaac tcctgaccct
gtgatccgcc cgcctcggcc tcccaaagtg ctgggattac aggcatgagc cactgcaccc
ggccctgaga atatatttat taaagccacc tcttcactga aagttaccga aagagtcggt
ttaggaagga aacgaagggt cagtgaacag agtcaaatgc agaagtgggc ttgtcatggg
tagggctttc ggcgtacgat aaaaggatca tttgtttttt aaaaggggtt ggaaaaactg

gttttccagt tggaaacagt aaaggttgta agctttgtgt gtacaaaaga aaacagggaa
tgcaggtgtg tttatagcgt tgtggttcaa gtccctctta acaagaactc caaagctgga
aagcaggagg gaacaaaggt gaacatgaag gcgaggatgc tggggccctg cagtgcgctc
taggctgtgc gtgagccggg actgtaccca cagcttgctg agggctgctc ttcttgggcc
agggaaagca gggcagccgg gacctgcggc tgtgcctgga ctgaagctgt cccgcaggtc
cccaccctcc aacacgtgct cacctgtccc cctcctcgca gcagcctcgg gacaaaacaa
tgactcaagg acagcacttc tcgcagaagg tctggaagtg cccagaatgg gaggcacgga
agcccctccc ggggaggact cccgcgttga tggaccgttc ttggtgcaga ctcctgactg
cgtgcatgaa acctgagaca agtgcaattc cttccatgtc gccccagagt gcccaggagg
caggcagtgc ggggtgccca ggcagacggg ttcagcctgc agaactggag gcgacctgtg
aaacccaccc gggcacccca acaggaacag aagcgtggtc ctgcggctgc gtccccagcg
agtttcactt tccccttgct cgtttctccc ttgttgtaag tgtttacaac tggcatgtgc
ttttaaacgt caggtaagag gggaacagct gctgtacatc gtcctggcga gtgacaatgt
gacagaagcc tgggcgaggc cctcggaggg cagcagctgg acaggggcta ctgggtttgg
cctggacagc actgatttgt ggatgtggat gggggcacgt tgtccgtgat aaaagtacaa
gtgcccctca caaaaaaaaa aaaaaaaa (SEQ ID NO:1; Underlined: the coding sequence)

mrlssspprg pqqlssfgsv dwlsqsscsg pthtprpadf slgslpgpgq tsgareppqa
vsikeaagss nlpapertma glskepntlr aprvrtaftm eqvrtlegvf qhhqylsple
rkrlaremql sevqiktwfq nrrmkhkrqm qdpqlhspfs gslhappafy stssglangl
qllcpwapls gpqalmlppg sfwglcqvaq ealasagasc cgqplashpp tpgrpslgpa
lstgprglca mpqtgdaf (SEQ ID NO:2; Underlined: homeodomain)

Described herein is a method of treating a cancer in a subject by administering to the subject macrophages that exhibit anti-tumor activities. As used herein, unless otherwise specified, the terms "macrophages that exhibit anti-tumor activities," "M1-like macrophages," and "macrophages that exhibit an M1 phenotype" may be used interchangeably.

M1-like macrophages can be produced by (1) increasing Hom-1 expression in macrophages or monocytes, (2) increasing expression of one or more M1 genes in macrophages or monocytes, and/or (3) inhibiting expression of one or more M2 genes in macrophages or monocytes.

Monocytes (e.g., derived from a subject's peripheral blood) can be used in the method as it was shown that Hom-1 expression is both necessary and sufficient for monocyte-to-macrophage differentiation. In other words, increasing the expression of Hom-1 in monocytes can drive them to differentiate to macrophages.

Macrophages or monocytes can be induced *ex vivo* to express a higher level of endogenous Hom-1. Various agents or treatments can be used to induce Hom-1 expression, e.g., LPS, cholera toxin (CTX), chemotherapeutic agents, radiation, cytokines (e.g., GM-CSF), phorbol 12-myristate 13-acetate (PMA), and antibodies or RNAi against inhibitors of Hom-1 expression.

Macrophages or monocytes that have been genetically modified to express an elevated level of Hom-1 can also be used to treat a subject with cancer. For example, the genetically modified macrophages or monocytes can contain a nucleic acid sequence encoding a Hom-1 polypeptide or a fragment thereof that includes the Hom-1 homeobox domain. The nucleic acid sequence is operably linked to a promoter and the modified macrophages or monocytes express the Hom-1 polypeptide or the fragment thereof. Such modified macrophages or modified monocytes (which would differentiate to macrophages) express a sufficiently high level of Hom-1 to exhibit anti-tumor activities and/or an M1 phenotype.

Genetically modified macrophages or monocytes can also be generated by introducing extra copies of the Hom-1 gene into the macrophages or monocytes. For example, an expression construct containing a Hom-1 nucleic acid sequence (encoding a Hom-1 polypeptide or a fragment thereof that includes the Hom-1 homeobox domain) operably linked to the endogenous Hom-1 promoter can be introduced into macrophages or monocytes.

A Hom-1 polypeptide or fragment thereof that includes the Hom-1 homeobox domain can also be introduced into macrophages or monocytes by direct peptide delivery.

Methods known in the art can be used to genetically modify macrophages and monocytes. For example, an exogenous expression construct for expressing Hom-1 can be introduced (e.g., stably or transiently transfected into) macrophages or monocytes. In one embodiment, the Hom-1 nucleic acid sequence is operably linked to a heterologous (i.e., not a Hom-1 promoter) constitutive or inducible promoter. In one embodiment, the Hom-1 nucleic acid sequence is operably linked to an endogenous promoter.

M1-like tumoricidal macrophages can also be generated by inducing expression of M1 genes in macrophages or monocytes. Agents that can induce M1 genes include, but not limited to, LPS, CTX, PMA, GM-SCF, INFγ, and chemotherapeutic agents. Macrophages or monocytes can also be genetically modified to express elevated levels of M1 genes. M1 genes include IL1b, IL6, IL12, IL23, TNFα, iNOs, CD40, CD80, CD86, CD68, TLR4, TLR2, IL-1R, MHCII, CCL15, CCL20, CXCL9, CXCL1, and SOCS3.

Inhibiting expression of M2 genes in macrophages or monocytes can also produce M1-like tumoricidal macrophages. Agents that inhibit M2 genes include anti-IL4 agents (e.g., antibodies or RNAi agents), anti-IL13 agents (e.g., antibodies or RNAi agents), antibodies against M2 proteins, and RNAi agents targeting M2 genes. M2 genes include ARG1, MMP9, CCL18, VEGF, IL10, IL4, TGFb, CD163, CD206, CD68.,TLR8, TLR1, MHCII, TGM2, DcoyR, IL-1RII, Ym1/2, MMR/CD206, and SR.

Heterologous or autologous macrophages or monocytes can be used to generate M1-like macrophages. If heterologous macrophages or monocytes are used, HLA-matching can be conducted to avoid or minimize host reactions. HLA un-matched macrophages or monocytes may also be used. Autologous macrophages or monocytes can be obtained from a cancer patient using methods known in the art.

The generated M1-like macrophages can be administered to a subject through infusion or injection (for example, via intravenous, intrathecal, intramuscular, intraluminal, intratracheal, intraperitoneal, intracranial, subcutaneous, or another type of intra tissue route), transdermal administration, or other routes known in the art. In one example, the macrophages or monocytes can be directly injected at a site or into a tissue (e.g., liver or pancreas) or its surrounding area, where a tumor is found.

The subject can be treated with the M1-like macrophages as often (e.g., every 1 to 30 days) and as many times (e.g., 1-30 times) as needed to treat the cancer. The M1-like macrophages described herein can also be used in a combination therapy with other cancer treatments such as radiation, chemotherapy, antibodies, and small molecules drugs.

The data described below show that Hom-1 expression converts TAMs into tumoricidal cells independent of tumor types. Hence, any cancer can be treated using the M1-like macrophages described herein, particularly cancers associated with TAMs that express a low level of Hom-1. Before treating a subject with M1-like macrophages as described above, it may be useful to determine whether the Hom-1 expression level in TAMs in the subject is lower than that found in a control (e.g., a macrophage found in a normal tissue in the subject).

Examples of cancer that can be treated with M1-like macrophages include, but are not limited to, carcinoma and sarcoma such as leukemia, sarcoma, osteosarcoma, lymphomas, melanoma, glioma, glioblastoma, pheochromocytoma, hepatoma, ovarian cancer, skin cancer, testicular cancer, gastric cancer, pancreatic cancer, renal cancer, breast cancer, prostate cancer, colorectal cancer, cancer of head and neck, brain cancer, esophageal cancer, bladder cancer, adrenal cortical cancer, lung cancer, bronchus cancer, thyroid cancer, endometrial cancer, nasopharyngeal cancer, cervical cancer, liver cancer, metastatic cancer, and cancer of unknown primary site.

Detecting a lower expression level of Hom-1 or an M1 gene or a higher expression level of an M2 gene in macrophages found in the microenvironment of a tissue area as compared to that of a control (e.g., a corresponding level in a macrophage in a normal tissue) indicates that the tissue area is a cancer or is at risk of becoming a cancer. Thus, also contemplated herein is a method for identifying whether a suspected tissue area is a cancer or at risk of becoming a cancer.

Further described herein are methods of identifying a candidate compound for treating cancer. A test cell (i.e., a macrophage or monocyte) can be contacted with a test compound and the expression level of (i) Hom-1, (ii) a reporter gene operably linked to Hom-1 promoter, (iii) an M1 gene, (iv) a reporter gene operably linked to an M1 promoter, (v) an M2 gene, or (vi) a reporter gene operably linked to an M2 promoter in the test cell is detected. A test compound that increases the expression level of any of (i)-(iv), and/or decreases the expression level of (v) or (vi) as compared to a control (e.g., a corresponding level in a test cell not contacted with the test compound) is a candidate compound for treating cancer.

In one screening method, a test compound is added to a co-culture containing a test cell and a cancer sample. A test compound is a candidate compound for treating cancer if it, as compared to a control, (i) increases the expression level of Hom-1, a reporter gene operably linked to a Hom-1 promoter, an M1 gene, or a reporter gene operably linked to an M1 promoter in the test cell, (ii) inhibits a significant decrease of the expression level of Hom-1, a reporter gene operably linked to a Hom-1 promoter, an M1 gene, or a reporter gene operably linked to an M1 promoter in the test cell, or (iii) decreases the expression level of an M2 gene or a reporter gene operably linked to an M2 promoter in the test cell.

In a co-culture system, whether a test compound has an inhibitory effect on the cancer sample can also be determined. A test compound that inhibits the cancer sample (e.g., inhibits growth of a cancer cell, kills a cancer cell, or decreases the size of the cancer sample) as compared to a control is a candidate compound for treating cancer. The test cell and the cancer sample can be in direct contact with each other. Alternatively, the test cell and the cancer sample are not in direct contact (e.g., with the use of transwell inserts). The cancer sample can be a sample containing a cancer cell, for example, a cancer tissue sample, a cancer cell isolated from a cancer tissue sample, or a cell of a cancer cell line. Cancer tissue samples can be obtained from surgically dissected specimens from cancer patients. Such cancer tissues samples may contain TAMs.

A screening method can also be performed with a cancer tissue sample in the absence of a test cell. A cancer tissue sample can be contacted with a test compound. After a time period, TAMs can be isolated from the cancer tissue sample and the expression level of Hom-1, an M1 gene, or an M2 gene in the TAMs can be determined. Alternatively or in addition to, the expression level of Hom-1 in the tissue sample can be determined. A test compound is a candidate compound for treating cancer if it, as compared to a control, (i) increases the expression level of Hom-1 or an M1 gene, (ii) inhibits a significant decrease of the expression level of Hom-1 or an M1 gene, and/or (iii) decreases the expression level of an M2 gene. A test compound that inhibits the cancer tissue sample (e.g., decreases the size of the sample) as compared to a control is also considered as a candidate compound for treating cancer.

Test compounds to be screened (e.g., proteins, peptides, peptidomimetics, peptoids, antibodies, RNAi, small molecules, or other drugs) can be obtained using a method known in the art.

In any of the methods described herein, the expression level of Hom-1, an M1 gene, or an M2 gene can be determined at either the mRNA level or at the protein level. Promoter activities can also be measured. Methods of measuring mRNA levels, protein levels, and promoter activities are well known in the art.

In any of the above-described screening methods, the test cell can be a macrophage or monocyte. The macrophage can be an M1 macrophage, an M2 macrophage, a tumor-associated macrophage, a tissue macrophage, or a monocyte-derived macrophage. The test cell can also be a monocyte.

The following are numbered statements of invention:
1. A method of treating a cancer, the method comprising:
   providing a genetically modified macrophage or monocyte that contains a nucleic acid sequence encoding a Hom-1 polypeptide or a fragment thereof that contains the Hom-1 homeobox domain, wherein the nucleic acid sequence is operably linked to a heterologous promoter and the modified macrophage or monocyte expresses the Hom-1 polypeptide or the fragment thereof; and
   administering the modified macrophage or monocyte to a subject with a cancer.
2. The method of statement 2, wherein the modified macrophage or monocyte is generated by introducing an exogenous expression construct into a macrophage or monocyte derived from the subject or another subject.
3. The method of statement 1 or 2, wherein the promoter is a constitutive promoter.
4. The method of statement 1 or 2, wherein the promoter is an inducible promoter.
5. The method of statement 1, wherein the modified macrophage exhibits an M1 phenotype.
6. The method of any of statements 1-5, further comprising, prior to the administering step, detecting a lower level of Hom-1 expression in a tumor-associated macrophage in the subject as compared to a control.
7. A method of treating a cancer, the method comprising:
   contacting a macrophage or monocyte with one or more agents that induce expression of Hom-1, whereby the expression level of endogenous Hom-1 in the macrophage or monocyte is higher than before the contacting step; and
   administering the thus contacted macrophage or monocyte to a subject with a cancer.
8. The method of statement 7, wherein the macrophage or monocyte is autologous or heterologous to the subject.
9. The method of statement 7, wherein the contacting step and the administering step are repeated at least once.
10. A method of treating a cancer, the method comprising:
   contacting a macrophage or monocyte with an agent that induces the expression of an M1 gene or an agent that inhibits the expression of an M2 gene, whereby a macrophage that exhibits an M1-phenotype is generated; and
   administering the thus generated macrophage to a subject with cancer.
11. The method of statement 10, wherein the macrophage or monocyte is autologous or heterologous to the subject.
12. A method of identifying a cancer in a subject, the method comprising:
   detecting the expression level of a gene in a macrophage from the microenvironment of a tissue area suspected of being a cancer, the gene being a Hom-1 gene, M1 gene, or M2 gene, wherein detecting (i) a lower level of the Hom-1 gene or M1 gene or (ii) a higher expression level of the M2 gene as compared to a corresponding control level indicates that the suspected tissue area is a cancer or is at risk of becoming a cancer.
13. A method of identifying a candidate compound for treating a cancer, the method comprising:
   contacting a test cell with a test compound, wherein the test cell is a macrophage or monocyte;
   detecting in the test cell the expression level of (i) Hom-1, (ii) a reporter gene operably linked to a Hom-1 promoter, (iii) an M1 gene, or (iv) an M2 gene; and
   selecting a test compound that alters the expression level as compared to a corresponding control, wherein the selected compound is a candidate compound for treating cancer.
14. The method of statement 13, further comprising contacting a protumor macrophage with the selected compound and assaying the thus contacted protumor macrophage for an anti-tumor activity.
15. The method of statement 13 or 14, wherein the protumor macrophage is a tumor-associated macrophage or M2 macrophage.
16. The method of statement 13, wherein the contacting step is performed with the test cell in a co-culture containing a cancer sample.
17. The method of statement 16, wherein the test cell and the cancer sample are in direct contact or in indirect contact with each other.
18. The method of statement 16, wherein the test cell is an M1 macrophage, an M2 macrophage, a tumor-associated macrophage, a tissue macrophage, or a monocyte-derived macrophage.
19. A method of identifying a candidate compound for treating a cancer, the method comprising;
   providing a co-culture containing a test cell and a cancer sample, wherein the test cell is a macrophage or monocyte;
   adding a test compound to the co-culture; and
   selecting a test compound that, as compared to a corresponding control, (i) inhibits the cancer sample, (ii) increases the expression level of Hom-1 or a reporter gene operably linked to a Hom-1 promoter in the test cell, (iii) increases the expression of an M1 gene in the test cell, (iv) decreases the expression of an M2 gene in the test cell, or (v) inhibits a significant decrease of the expression level of Hom-1 or a reporter gene operably linked to a Hom-1 promoter in the test cell;
   wherein the selected compound is a candidate compound for treating cancer.
20. The method of statement 19, wherein the cancer sample is a cancer tissue sample.
21. The method of statement 19 or 20, wherein the test cell and cancer sample are in direct contact or in indirect contact with each other.
22. The method of statement 19, wherein the test cell is an M1 macrophage, an M2 macrophage, a tumor-associated macrophage, a tissue macrophage, or a monocyte-derived macrophage.

The specific example below is to be construed as merely illustrative, and not limitative of the remainder of the disclosure in any way whatsoever. Without further elaboration, it is believed that one skilled in the art can, based on the description herein, utilize the present disclosure to its fullest extent. All publications cited herein are herein incorporated by reference in their entirety.

### EXAMPLE

We identify a role of the human homeobox protein Hom-1 in functional polarization of human macrophages. We found that Hom-1 promotes and is required for M1 activation of human macrophages, but not required for the expression of critical genes involved in M2 activation.

Using primary TAMs isolated from cancers, we found that Hom-1 expression in TAMs was significantly decreased in comparison with macrophages isolated from normal control tissues. We showed that the expression profile of Hom-1 in TAMs correlated with TAM phenotypes. Moreover, ectopic expression of Hom-1 converted TAMs into M1-like phenotype. Both *in vitro* and *in vivo* data showed that Hom-1 conferred tumoricidal activity to TAMs. Taken together, our studies demonstrated that Hom-1 converted TAMs into tumoricidal cells. We showed that Hom-1-expressing TAMs (exhibiting an M1 phenotype) exerted strong inhibitory effects on the growth of a variety of cancers, suggesting the role of Hom-1-modulated TAMs as a new modality in the treatment of cancers.

### Hom-1 expression was decreased in TAMs

In advanced tumors, TAMs display a protumor M2-like phenotype. See, Bronte and Murray (2015), Nat Med 21, 117-119. The plasticity of TAMs has been well appreciated, and a variety of cytokines have been implicated in the polarization of TAMs towards M2 phenotype. See, Noy and Pollard (2014), Immunity 41, 49-61. In comparison, the transcriptional machinery that controls the TAM polarization remains largely unknown.

Discarded surgical specimens from colon cancer resections were used to isolate TAMs from tumor tissues as well as macrophages from normal mucosa 15 cm away from the tumor sites as described below. As previously noted, FACS analysis showed that, in comparison to macrophages isolated from normal control mucosa, TAMs expressed significant higher levels of cell surface markers associated with M2 phenotypes, such as the CD68, CD163, CD206. See, Zhang et al. (2013), Eur J Cancer 49, 3320-3334. We found that there was no significant difference in the expression of non-discriminating macrophage marker CD33 in TAMs and control macrophages.

To determine whether Hom-1 may play a role in TAMs, we quantified Hom-1 expression in TAMs by qRT-PCR and found that, in comparison with its expression in control macrophages, Hom-1 expression is significantly decreased in TAMs.

### VentX regulated TAM plasticity and polarized TAMs toward an M1 phenotype

Previous studies indicated that TAMs can be induced by LPS to display an M1 phenotype. See, Zhang et al. To determine whether Hom-1 plays a role in TAM plasticity, we examined Hom-1 expression in TAMs exposed to LPS. We found that Hom-1 expression was significantly elevated in TAMs after being stimulated with LPS. Parallel with the elevated expression of Hom-1 and consistent with prior findings, LPS stimulation of TAMs led to elevated secretion of inflammatory cytokines and cytotoxic iNOs.

To determine whether Hom-1 plays a regulatory role of TAM plasticity, we examined the effects of Hom-1 knockdown on TAM phenotype. Treatment of TAMs with anti-Hom-1 morpholinos (MO) led to around an 80% reduction of Hom-1 expression. Consistent with Hom-1's role as a key regulatory of TAM plasticity, we found that Hom-1 MO aborted LPS induced secretion of inflammatory cytokines and cytotoxic iNOs in TAMs. CD206 is a mannose receptor and a M2 cell surface marker that is highly expressed in TAMs. Reflecting the plasticity of TAMs, exposure of TAMs to LPS led to a significant reduction of CD68+CD206+ population and an increase of the CD68+CD206- cell numbers. Hom-1 MO abolished both effects of LPS on TAMs (p < 0.01).

The correlation between Hom-1 expression levels and TAM phenotypes prompted us to further explore the idea that Hom-1 controls TAM plasticity. TAMs were isolated and transfected with a plasmid encoding GPF-Hom-1 or control GFP. Compared with the control GFP transfected TAMs, TAMs transfected with GFP-Hom-1 displayed characteristics M1 morphology with elongated/fibroblast-like cell shape. FACS analysis showed that surface expressions of M1 markers CD40, CD80, and CD86 were significantly increased in TAMs transfected with GFP-Hom-1. In addition, secretions of pro-inflammatory cytokines TNFα, IL-1ß, and IL-12 were significantly increased, while secretion of M2 cytokine IL-10 was significantly decreased in TAMs transfected with GFP-Hom-1. Consistently, gene expression analysis showed that M1 genes, such as IL-1ß, IL-6, TNF-α, and iNOs increased significantly in TAMs transfected with GFP-Hom-1, while M2 genes, such as, CCL18, MMP9, VEGFA, and Arg1 decreased significantly in TAMs transfected with GFP-Hom-1. Taken together, our data suggested that Hom-1 regulated TAM plasticity and promoted M1 polarization of TAMs.

### Hom-1 converted TAMs into tumor suppressing cells

Our findings that Hom-1 promotes M1 polarization of TAMs prompted us to explore whether Hom-1-modified TAMs could exert tumor suppression. Freshly isolated TAMs from colon cancers were transfected with plasmid encoding GPF-Hom-1 or control GFP. The modified TAMs were then co-cultured with tumor or normal tissues of the same patient, using the trans-well culture system. Remarkably, after 7-10 days of co-culturing, tumor volumes were significantly decreased (around 70%) during the incubating with GFP-Hom-1 modified TAMs (p <0.01), while there was no significant change of size in tumors incubated with GFP-transfected TAMs or with TAMs alone. To determine whether the shrinkage of tumor volume was related to reduction of cancer cells, we performed tissue section, H&E staining and immunohistochemistry of colon cancer cells with CK20 antibody. We found that CK20 positive tumor cells appeared in nests, cords, and sheets in the tumors incubated with TAMs or GFP modified TAM. However, CK20 positive tumor cells disappeared during the incubation with TAMs transfected with GFP-Hom-1. The specificity of tumoricidal effects of Hom-1-modified TAMs was demonstrated by the findings that Hom-1-modified TAMs exerted minimal effects on the volume and morphology of normal colon mucosa during the incubation with TAMs transfected with either GFP or GFP-Hom-1.

### Hom-1 promotes tumoricidal function of TAMs in vivo

Our findings that Hom-1 converted TAMs into tumoricidal cells *in vitro* prompted us to explore the potential role of Hom-1-regulated TAMs in tumorigenesis *in vivo.* Colon cancers were cut into around 0.5 cm pieces and surgically seeded in the subcutaneous space of the abdominal side of NSG mice. One week later, MO-Hom-1 transfected TAMs (Hom-1 inhibited) or GFP-Hom-1 transfected TAMs (Hom-1 expressing) were injected through mouse tail vain. After 8 weeks of xenograft, tumors developed in mice injected with MO-Hom-1 transfected TAMs, but not in mice injected with GFP-Hom-1 transfected TAMs.

We also found that the TAMs or monocytes can be induced to exhibit an M1 phenotype by culturing them in an M1 differentiation media. These M1-differentiated TAMs/monocytes can be infused into NSG mice and inhibit cancer growth *in vivo.* The effects of M1 differentiated TAMs on tumor growth are abolished by inhibition of Hom-1 expression in these TAMs or monocytes.

### Effects of ectopic expression of Hom-1 in TAMs on various cancer types

TAMs have been implicated in oncogenesis of essentially all tumors. Following our studies on TAMs in colon cancer cells, we extended our investigation to other tumor types.

Surgical species of lung, melanoma, esophagus, gastric, and pancreatic cancers were obtained, and TAMs were isolated as described above. Macrophages from corresponding normal tissues of the same patient were obtained. Hom-1 expression in TAMs and tissue macrophages were quantified using real time RT-PCR. Hom-1 expressions in TAMs of all these tumors were low in comparison to Hom-1 expression in corresponding macrophages from distant normal tissues.

To determine whether Hom-1 could convert these TAMs into tumoricidal cells, GFP or GFP-Hom-1 were transfected into the TAMs. After 48 hours of transfection, GFP positive cells were sorted out and co-cultured with individual tumors. Tumor volumes of all tumors decreased during co-culture with GFP-Hom-1 transfected TAMs but not with the control GFP-transfected TAMs. Our results suggested that Hom-1 could convert TAMs into tumoricidal cells independent of tumor types.

### Collection of colon tissue samples

Cancer tissues and normal tissues were obtained from surgically dissected specimens from patients in pathology lab. Around 5-10 gram of tissue was collected from each tumor mass, or from normal mucosa at 15 cm away from tumor mass. Patient blood samples were also collected.

### Preparation of intraepithelial lymphocyte

Lamina propria mononuclear cells (LPMCs) were isolated using previously described techniques with modification (Kamada N, et al, 2008; Pignata C, et al, 1990). In brief, dissected fresh mucosa and tumor mass were rinsed in 10-cm Petri dish with Ca²⁺-free and Mg²⁺-free hank's balanced salt solution (HBSS) (life technologies) containing 2% fetal bovine serum (FBS) and 1 mM Dithiothreitol (DTT) (Sigma-Aldrich) to remove mucus. The mucosa and tumor were cut into 0.5 cm pieces by a razor blade and incubated in 6-well plate with 5 mL HBSS containing 1 mM EDTA (Sigma-Aldrich) at 37°C for 1 hour, then passed through a gray-mesh (100 micron). The flowthrough contains intraepithelial lymphocyte and epithelial cells and was analysis by a flow cytometer.

### Isolation of macrophage from tumor mass and normal mucosa

Subsequently, the mucosa and tumor were incubated in HBSS (with Ca²⁺ and Mg²⁺), containing 2% FBS, 1.5 mg/mL Collagenase D (Roche), 0.1 mg/mL Dnase I at 37°C for 1 hour. Digested tissues were passed through a gray-mesh (70 micron) filter. The flowthrough was collected and resuspended in a 40% Percoll solution (Pharmacia), then layered on 60% Percoll, and centrifuged at 2000 rpm for 30 min without brake. LPMCs at the interface were collected. Normal mucosal macrophages and TAMs were purified from LPMCs using Easy Sep^{™} Human Monocyte/Macrophage Enrichment kit without CD16 depletion (StemCell Technologies), according to the manufacturer's instructions. Cells isolated by these techniques were routinely more than 98% viable by propidium iodide (PI) staining. The purity of intestinal macrophages was more than 95%.

### Preparation of macrophages from peripheral blood

Peripheral blood mononuclear cells (PBMC) from healthy adult donors at Brigham and Women hospital were isolated by Ficoll density gradient centrifugation. Human monocytes were purified from PBMCs using EasySep^{™} Human Monocyte Enrichment kit without CD16 depletion according to the manufacturer's instructions. Purified cells were cultured in completed RPMI medium with 10 ng/mL of M-CSF (PeproTech). After enrichment, monocytes were cultured in completed RPMI medium with M-CSF for 5 days, cells were used for co-culture system.

### FACS analysis

Phenotypic analysis of TAMs and other lymphocytes was performed using flow cytometry after immunolabeling of cells with fluorescence dye-conjugated antibodies. The following antibodies were used: PE-conjugated anti-CD3 (OKT3), -CD25 (BC96), -CD14 (61D3), -CD68 (eBio Y182A), -CD163 (eBio GH161), -CD206, FITC-conjugated anti-CD4 (RPA-T4), -CD33 (HIM3-4), APC-conjugated anti-CD8 (OKT8), -CD4 (OKT4) (eBioscience, Inc). Intracellular staining of Foxp3 (236A/E7), IFN-γ, Perforin, and Granzyme B was performed with PE-conjugated antibodies following the protocol provided by manufacturer. Isotope control labeling was performed in parallel. Antibodies were diluted as recommended by the supplier. Labeled cells were collected on FACScan flow cytometer with Cell-Quest software (BD Biosciences) and analyzed by FlowJo software. Results are expressed as the percentage of positive cells.

### Organotypic co-cultures of tumor and macrophages

Transwell inserts (0.4 µm pore sized, Costar, Corning) were placed in 12-well polystyrene tissue culture plates (Becton Dickinson, Franklin Lakes, NJ). Mucosa and tumor mass were weighed and washed with 1x PBS buffer, plus antibiotics, then cut into 0.5 cm pieces. Around 50 mg of tissues were seeded in the upper compartment of a 12-well transwell and filled with 0.5 mL of RPMI 1640 completed medium. 5 x 10⁵ of TAMs were added to the lower compartment at the density of 0.5 million cells/well with no direct cell-tissue contact and filled with 2 mL of PRMI completed medium. The plate incubated at 37°C, 5% CO₂. 0.5 mL of culture medium were collected for cytokine analysis and fresh medium were added every three days. After two weeks co-culture, low chamber macrophages were collected and total RNA was isolated by the TRIzol reagent (Ambion). Tumor and normal mucosal were monitored two times a week by calipers to calculate tumor volumes according to the formula (length x width²) / 2. The photos of tissues were taken by a 3-megapixel CMOS camera on Leica EZ4D stereomicroscope or a digital camera on iPhone.

### Immunohistochemistry

Tumors or normal tissues were fixated in Formalin (Fisher Scientific Company, Kalamazoo, MI). CK20 stainings (Dako, Carpinteria, CA, clone Ks20.8, 1:50) and Haematoxylin/eosin (H&E) stainings were performed. CK20 staining was performed on the Leica Bone III staining platform using Epitope Retrieval 2 for 20 minutes online, and using Bone Polymer Refine detection kit. Microscopic analysis was performed with a Nikon Eclipse Ti fluorescence microscopy. Images were captured at an original magnification of 40 x using a color camera applying the NIS Elements imaging software (Nikon). Brightness and contrast for representative images were adjusted equally among groups.

### Hom-1 overexpression

Transfection of GFP-Hom-1 into blood macrophages and TAMs was carried out through lipofectamine 2000 (Life technologies) according to manufacture protocol. 48 hours after transfection, cells were filtered through a 70 um filter for cell sorting. GFP positive cells were sorted by BD FACSAria II under the Baker Bio-Protect Hood in a sterile condition. After sorting, cells were cultured in RPMI 1640 completely medium.

### Hom-1 knockdown

Colon TAMs or human primary monocytes were transfected with Morpholino (MO) antisense oligonucleotides using the Human Monocyte Nucleofector Kit (Lonza, Walkersville, MD) according to the manufacturer's instructions. Briefly, 5 × 10⁶ cells were re-suspended into 100 µl nucleofector solution with 2.5 nmol of either Hom-1 MO oligonucleotides or a standard control MO oligonucleotides and electroporated with the Nucleofector II Device (Lonza). Cells were then immediately removed from the device and incubated overnight with 1ml pre-warmed Human Monocyte Nucleofector Medium containing 2mM glutamine and 10% FBS. Cells were then re-suspended into complete RPMI medium and treated with appropriate cytokines to induce differentiation into macrophages. All the MO oligonucleotides were ordered from Gene Tools (Philomath, OR).

### Cytokine measurement

Levels of IL-1β, IL-10, TNF-α and IL-12p70 in the supernatants of *E. coli* LPS-(Sigma-Aldrich) treated blood macrophages or LPS-treated TAMs were quantified using ELISA kits obtained from eBiosciences. Analyses were conducted according to the manufacturer's instructions.

### qRT-PCR

Total RNA was isolated by the TRIzol reagent and RNA amounts were measured by NanoDrop 2000 (Thermo Scientific). An equal amount of RNA was used for first-strand cDNA synthesis with SuperScript III First-Strand Synthesis System (Life Technologies) according to the manufacturer's protocol. To amplify Hom-1 cDNA with conventional PCR, we used the AccuPrime Taq DNA polymerase system (Life Technologies) following the manufacturer's instructions. PCR products were separated on 2% agarose gels and stained with ethidium bromide. GAPDH was used as an internal control. We performed quantitative measurement of Hom-1 and other genes cDNA with SYBR Green on a LightCycler (480 Real-Time PCR System; Roche). Relative expression profiles of mRNAs were then calculated using the comparative Ct method (DDCT method).

### Arginase activity and NO assays

Arginase activity was quantified in cell lysates by measuring the production of urea using the QuantiChrom arginase Assay Kit (DARG-200; BioAssays Systems). Nitrite concentrations in culture supernatants were determined using Griess reagent kit (Molecular Probes).

### Statistical Analysis

Student's test was used for statistical analysis.

### OTHER EMBODIMENTS

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features.

From the above description, one skilled in the art can easily ascertain the essential characteristics of the described embodiments, and without departing from the spirit and scope thereof, can make various changes and modifications of the embodiments to adapt it to various usages and conditions. Thus, other embodiments are also within the claims.

## Claims

1. A method of identifying a cancer in a subject, the method comprising: detecting the expression level of a gene in a macrophage from the microenvironment of a tissue area suspected of being cancer, the gene being a Hom-1 gene, M1 gene, or M2 gene, wherein detecting (i) a lower level of the Hom-1 gene or M1 gene, or (ii) a higher expression level of the M2 gene as compared to a corresponding control level indicates that the suspected tissue area is cancer or is at risk of becoming cancer.

2. The method of claim 1, wherein the expression level of said Hom-1 gene, said M1 gene, or said M2 gene is determined at the mRNA level.

3. The method of claim 1, wherein the expression level of said Hom-1 gene, said M1 gene, or said M2 gene is determined at the protein level.

4. The method of any one of claims 1-3, wherein said M1 gene comprises IL1b, IL6, IL12, IL23, TNFα, iNOs, CD40, CD80, CD86, CD68, TLR4, TLR2, IL-1R, MHCII, CCL15, CCL20, CXCL9, CXCL1, or SOCS3.

5. The method of any one of claims 1-4, wherein said M2 gene comprises ARG1, MMP9, CCL18, VEGF, IL10, IL4, TGFβ, CD163, CD206, CD68, TLR8, TLR1, MHCII, TGM2, DcoyR, IL-1RII, Ym1/2, MMR/CD206, or SR.

6. The method of any one of claims 1-5, wherein said cancer is associated with tumor-associated macrophages (TAMs) that express a lower level of Hom-1 than macrophages in a control or a normal tissue of the subject having the cancer.

7. The method of any one of claims 1-6, wherein said cancer is carcinoma, sarcoma, leukemia, osteosarcoma, lymphoma, melanoma, glioma, glioblastoma, pheochromocytoma, hepatoma, ovarian cancer, skin cancer, testicular cancer, gastric cancer, pancreatic cancer, renal cancer, breast cancer, prostate cancer, colorectal cancer, cancer of head and neck, brain cancer, esophageal cancer, bladder cancer, adrenal cortical cancer, lung cancer, bronchus cancer, thyroid cancer, endometrial cancer, nasopharyngeal cancer, cervical cancer, liver cancer, metastatic cancer, or a cancer of unknown primary site.

8. A method of identifying a candidate compound for treating a cancer, the method comprising:
contacting a test cell with a test compound, wherein the test cell is a macrophage or monocyte;
detecting in the test cell the expression level of (i) Hom-1, (ii) a reporter gene operably linked to a Hom-1 promoter, (iii) an M1 gene, or (iv) an M2 gene; and
selecting a test compound that alters the expression level as compared to a corresponding control, wherein the selected compound is a candidate compound for treating cancer.

9. The method of claim 13, further comprising contacting a protumor macrophage with the selected compound and assaying the thus contacted protumor macrophage for an anti-tumor activity;
optionally, the protumor macrophage is a tumor- associated macrophage or M2 macrophage.

10. The method of claim 9, wherein the contacting step is performed with the test cell in a co-culture containing a cancer sample;
optionally, the test cell and the cancer sample are in direct contact or in indirect contact with each other; or
optionally, the test cell is an M1 macrophage, an M2 macrophage, a tumor-associated macrophage, a tissue macrophage, or a monocyte-derived macrophage.

11. A method of identifying a candidate compound for treating a cancer, the method comprising;
providing a co-culture containing a test cell and a cancer sample, wherein the test cell is a macrophage or monocyte;
adding a test compound to the co-culture; and
selecting a test compound that, as compared to a corresponding control, (i) inhibits the cancer sample, (ii) increases the expression level of Horn- 1 or a reporter gene operably linked to a Hom-1 promoter in the test cell, (iii) increases the expression of an MI gene in the test cell, (iv) decreases the expression of an M2 gene in the test cell, or (v) inhibits a significant decrease of the expression level of Hom-1 or a reporter gene operably linked to a Hom-1 promoter in the test cell; wherein the selected compound is a candidate compound for treating cancer.

12. The method of claim 11, wherein the cancer sample is a cancer tissue sample.

13. The method of claim 11 or 12, wherein the test cell and cancer sample are in direct contact or in indirect contact with each other.

14. The method of claim 11, wherein the test cell is an M1 macrophage, an M2 macrophage, a tumor-associated macrophage, a tissue macrophage, or a monocyte-derived macrophage.
